Europäisches Patentamt

European Patent Office

Office européen des brevets

(11) Publication number: **0 378 692**
**A1**

## EUROPEAN PATENT APPLICATION
### published in accordance with Art. 158(3) EPC

(21) Application number: 89906465.3

(22) Date of filing: 06.06.89

(86) International application number:
PCT/JP89/00571

(87) International publication number:
WO 89/11831 (14.12.89 89/29)

(51) Int. Cl.⁵: **A61B 17/36, A61B 17/22**

(30) Priority: 06.06.88 JP 74913/88 U

(43) Date of publication of application:
25.07.90 Bulletin 90/30

(84) Designated Contracting States:
DE FR GB IT NL

(71) Applicant: SUMITOMO ELECTRIC INDUSTRIES, LTD
5-33, Kitahama 4-chome Chuo-ku
Osaka 541(JP)

(72) Inventor: SOGAWA, Ichiro Osaka Works of
Sumitomo Electr.
Ind., Ltd. 1-3, Shimaya 1-chome
Konohana-ku Osaka-shi Osaka 554(JP)
Inventor: NIWA, Shin-ichiro Osaka Works of
Sumitomo Electr.
Ind., Ltd. 1-3, Shimaya 1-chome
Konohana-ku Osaka-shi Osaka 554(JP)
Inventor: YOTSUYA, Koro Osaka Works of
Sumitomo Electr.
Ind., Ltd. 1-3, Shimaya 1-chome
Konohana-ku Osaka-shi Osaka 554(JP)
Inventor: UEMIYA, Takafumi Osaka Works of
Sumitomo Electr.
Ind., Ltd. 1-3, Shimaya 1-chome
Konohana-ku Osaka-shi Osaka 554(JP)
Inventor: KANAZAWA, Shin-ichi Osaka Works
of Sumitomi Electr
Ind., Ltd. 1-3, Shimaya 1-chome
Konohana-ku Osaka-shi Osaka 554(JP)

(74) Representative: Füchsle, Klaus, Dipl.-Ing. et al
Hoffmann . Eitle & Partner Patentanwälte
Arabellastrasse 4
D-8000 München 81(DE)

(54) CATHETER FOR MEDICAL TREATMENT.

(57) A catheter for a medical treatment, provided with a rotary blade at a portion in the vicinity of the front end thereof in such a manner that the rotary blade is partially exposed, and a laser beam guiding fiber

running up to the front end portion of the catheter body.

# Fig. 1

# D E S C R I P T I O N

Therapeutic Catheter

## TECHNICAL FIELD

The present invention generally relates to therapeutic catheters and more particularly, to a therapeutic catheter which is suitable for removing and treating diseased parts in vasa of living bodies, for example, blood vessels, oviducts, ureters, etc.

## BACKGROUND ART

In a known therapeutic catheter, a rotary blade is provided in the vicinity of a distal end of the catheter so as to be projected out of the catheter such that a stenosed part in a blood vessel, etc. are removed by the rotary blade.

In therapy employing the known catheter, the distal end of the catheter is initially guided to the stenosed part in the blood vessel and is passed through the stenosed part by dilating the stenosed part by the distal end of the catheter. At this time, thickened portions of the stenosed part are excised by the rotary blade disposed slightly rearwards of the distal end of the catheter such that the stenosed part is diminished.

However, in order to fulfil the above mentioned set object of therapy by using the known catheter, the distal end of the catheter is required to be thrusted through the stenosed part. Therefore, such a problem arises that the known catheter cannot be used in the case where the thickened portions closely obstruct the blood vessel so as to form a so-called obstructive part.

Furthermore, the known catheter has such an inconvenience that since excised tissue is stored in the catheter, the catheter is required to be drawn out of a living body as necessary and then, again inserted, if further therapy is necessary, into the living body after the stored tissue has been taken out of the catheter.

Meanwhile, such therapy is also performed in which diseased tissue obstructing a blood vessel completely is removed through its vaporization by irradiating laser beams thereto from a distal end of the catheter. However, since the diseased tissue is removed only through its vaporization, carbonized substance is likely to remain after irradiation of laser beams, so that thrombus readily adheres to the carbonized substance newly and thus, obstruction of the blood vessel is apt to take place again.

An object of the present invention is to provide, in view of the above described inconveniences, a therapeutic catheter which can remove diseased tissue of a completely obstructive part easily and enables therapy free from recurrence of obstruction through adherence of thrombus to the diseased tissue.

## DISCLOSURE OF INVENTION

In order to accomplish this object of the present invention, a therapeutic catheter according to the present invention is provided, in the vicinity of a distal end thereof, with a rotary blade such that said rotary blade partially projects out of said catheter, said catheter comprising: an optical fiber for transmitting laser beams, which is introduced into the distal end of said catheter.

By the above described arrangement of the therapeutic catheter, a portion of a completely obstructive part can be removed through its vaporization by irradiating laser beams such that a gap permitting passage of the distal end of the catheter therethrough can be formed at the completely obstructive part. Thus, the distal end of the catheter can be passed through the gap of the completely obstructive part. After the distal end of the catheter has been passed through the gap, the diseased tissue as well as carbonized substance produced at the diseased part upon irradiation of laser beams thereto can be removed by the rotary blade. Meanwhile, the excised tissue stored in a location inside the distal end of the catheter can be properly removed through its vaporization by an optical fiber for irradiating laser beams to the location. As a result, such an operation becomes unnecessary that the catheter is drawn out of a living body in order to remove the tissue stored in the catheter.

## BRIEF DESCRIPTION OF DRAWINGS

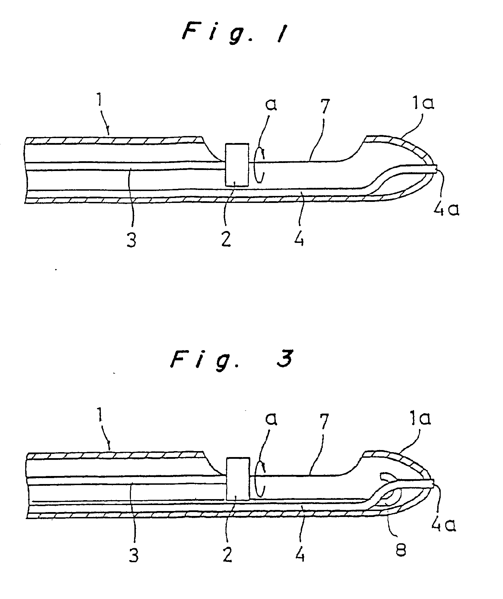

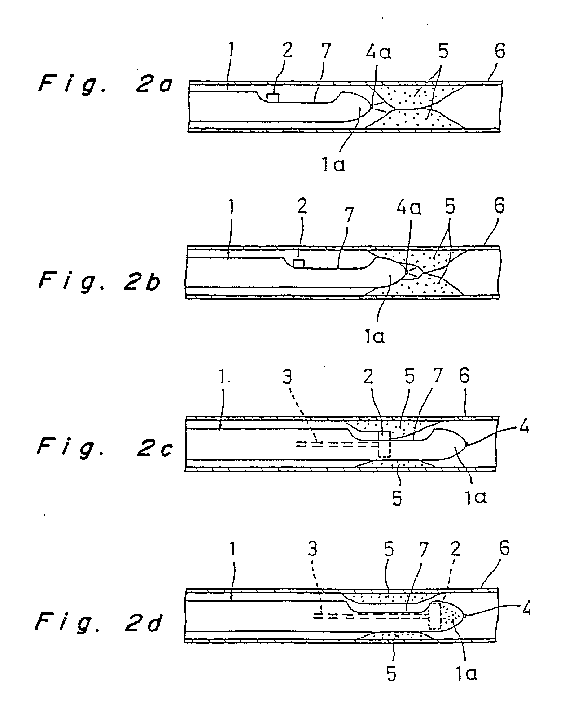

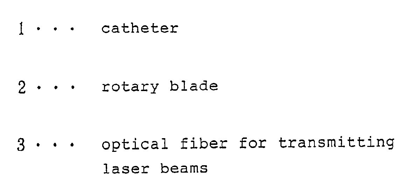

Fig. 1 is a longitudinal sectional view of a therapeutic catheter according to a first embodiment of the present invention, Figs. 2(a) to 2(d) are views explanatory of therapeutic steps employing this catheter and Fig. 3 is a longitudinal sectional view of a therapeutic catheter according to a second embodiment of the present invention.

## BEST MODE FOR CARRYING OUT THE INVENTION

Hereinbelow, embodiments according to the present invention will be explained by referring to the drawings. Fig. 1 is a sectional view of a therapeutic catheter according to a first embodiment of the present invention.

In Fig. 1, a distal end portion of the therapeutic catheter 1 (referred to as a "catheter", hereinbelow) is formed into streamlined shape so as to conveniently dilate a stenosed part, etc. when a distal end 1a of the catheter 1 is thrusted into the stenosed part, etc. The catheter 1 is cut out at its portion disposed rearwards of the distal end 1a so as to form a window 7 having a predetermined length in the axial direction of the catheter 1. A rotary blade 2 is provided at the window 7 and is movable towards the distal end 1a so as to partially project out of the window 7 while being rotated about a drive shaft 3 in the direction of the arrow a.

Furthermore, as an element characteristic of the present invention, an optical fiber 4 for transmitting laser beams is introduced into the catheter 1. A distal end 4a of the optical fiber 4 is so provided as to project forwards out of the distal end 1a of the catheter 1 or be flush with the distal end 1a.

Any optical fiber capable of transmitting power necessary for removing tissue of a diseased part through its vaporization can be employed as the optical fiber 4. However, as examples having a high breaking threshold value and easy to use, an optical fiber, in which at least its core is made of quartz such as pure quartz or quartz having Ge doped therein and its cladding is made of proper material such as quartz, glass, plastics or the like, can be selected. Meanwhile, any laser capable of removing the tissue of the diseased part through its vaporization, for example, excimer laser, YAG laser, argon laser, etc. can be employed.

As will be seen from Figs. 2a to 2d, in therapy, employing the catheter 1, the distal end 1a of the catheter 1 is initially guided to an obstructive part 5 in a blood vessel 6 (see Fig. 2a). Then, the distal end 1a is pressed against the obstructive part 5 (see Fig. 2b). At this time, laser beams are irradiated to the obstructive part 5 from the distal end 4a of the optical fiber 4. For a while, the catheter 1 is advanced while removing a portion of diseased tissue of the obstructive part 5 by irradiating laser beams thereto so as to dilate the obstructive part 5 such that the catheter 1 is passed through the obstructive part 5.

It is so arranged that when the distal end 1a of the catheter 1 has been passed through the obstructive part 5, a portion of the thickened diseased tissue of the obstructive part 5 is forced into the window 7. Subsequently, the rotary blade 2 is displaced towards the distal end of the catheter 1 while being rotated. Therefore, the portion of the obstructive part 5 is excised by the rotary blade 2 and thus, the catheter 1 is passed through the obstructive part 5 (see Fig. 2c). Then, the excised diseased tissue is collectively stored in a distal end portion of the catheter 1 (see Fig. 2d). Thereafter, in order to properly change location of the window 7 relative to the obstructive part 5, the catheter 1 is rotated through a predetermined angle and the above described operations are repeated.

Thus, by using the catheter of the above described arrangement, a portion of the obstructive part 5 which obstructs the blood vessel 6 completely can be removed through its vaporization, so that a gap permitting passage of the distal end 1a of the catheter 1 therethrough can be formed at the obstructive part 5.

Then, after the distal end 1a of the catheter 1 has been passed through the gap of the obstructive part 5, the diseased tissue and carbonized substance produced at the diseased part through irradiation of laser beams thereto can be removed by the rotary blade 2.

Furthermore, Fig. 3 shows a second embodiment. In addition to the first embodiment, this embodiment further includes an optical fiber 8 for irradiating laser beams to a portion for storing the excised tissue, which portion is disposed inside the distal end of the catheter. By using the optical fiber 8, the excised tissue stored inside the distal end of the catheter can be removed through its vaporization as necessary.

In accordance with the catheter of the present invention, a gap permitting passage of the distal end of the catheter therethrough can be formed at the completely obstructive part by irradiating laser beams thereto. Then, the diseased tissue and carbonized substance remaining at the diseased part can be removed by the rotary blade. Therefore, such a unique and practical effect can be achieved that the diseased tissue of the completely obstructive part can be removed easily and it becomes possible to perform therapy free from recurrence of obstruction of the blood vessel, etc. through adherence of thrombus thereto.

## C L A I M

1.      A therapeutic catheter which is provided, in the vicinity of a distal end thereof, with a rotary blade such that said rotary blade partially projects out of said catheter, said catheter comprising:

an optical fiber for transmitting laser beams, which is introduced into the distal end of said catheter.

Fig. 1

Fig. 3

EP 0 378 692 A1

Fig. 2a

Fig. 2b

Fig. 2c

Fig. 2d

1 · · ·  catheter

2 · · ·  rotary blade

3 · · ·  optical fiber for transmitting
laser beams

# INTERNATIONAL SEARCH REPORT

International Application No PCT/Jp89/00571

**I. CLASSIFICATION OF SUBJECT MATTER** (if several classification symbols apply, indicate all) 6

According to International Patent Classification (IPC) or to both National Classification and IPC

Int. Cl⁴ A61B17/36, 17/22

**II. FIELDS SEARCHED**

Minimum Documentation Searched 7

| Classification System | Classification Symbols |
|---|---|
| IPC | A61B17/36, 17/22 |

Documentation Searched other than Minimum Documentation
to the Extent that such Documents are Included in the Fields Searched 8

| | |
|---|---|
| Jitsuyo Shinan Koho | 1968 - 1989 |
| Kokai Jitsuyo Shinan Koho | 1971 - 1989 |

**III. DOCUMENTS CONSIDERED TO BE RELEVANT** 9

| Category * | Citation of Document, 11 with indication, where appropriate, of the relevant passages 12 | Relevant to Claim No. 13 |
|---|---|---|
| P | JP, A, 63-305857 (Olympus Optical Co., Ltd.) 13 December 1988 (13. 12. 88) Page 2, upper left column, line 16 to page 3, lower left column, line 7, Figs. 1 to 9 (Family : none) | 1 |
| Y | JP, A, 62-236537 (Olympus Optical Co., Ltd.) 16 October 1987 (16. 10. 87) Page 3, upper right column, line 1 to page 3, lower right column, line 4, Figs. 1 to 4 (Family : none) | 1 |
| Y | JP, A, 60-126171 (International Business Machines Corp.) 5 July 1985 (05. 07. 85) & EP, A, 144764 & DE, G, 3471875 | 1 |

\* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 8, 1989 (08. 08. 89) | August 28, 1989 (28. 08. 89) |

| International Searching Authority | Signature of Authorized Officer |
|---|---|
| Japanese Patent Office | |

Form PCT/ISA/210 (second sheet) (January 1985)